# EUROPEAN PATENT SPECIFICATION

(11) **EP 2 348 132 B1**
(45) Date of publication and mention of the grant of the patent: **04.12.2013**
(21) Application number: 10382010.6
(22) Date of filing: 21.01.2010
(51) Int. Cl.: C12Q 1/68, G01N 33/53, G01N 33/543

(54) **Method for the bioanalysis of nucleic acid molecules in a sample and biosensor for its implementation**
Verfahren zur Bioanalyse von Nukleinsäuremolekülen in einer Probe und Biosensor für dessen Durchführung
Procédé pour la bioanalyse de molécules d'acide nucléique dans un échantillon et biocapteur pour sa mise en oeuvre

(43) Date of publication of application: 27.07.2011
(73) Proprietor: CONSEJO SUPERIOR DE INVESTIGACIONES CIENTIFICAS, 28006 Madrid (ES)
(72) Inventor: Monteiro Kosaka, Priscila, 28001 Madrid (ES); Mertens, Johann, 28020 Madrid (ES); Tamayo de Miguel, Francisco Javier, 28760 Tres Cantos (Madrid) (ES); Calleja Gómez, Montserrat, 28760 Tres Cantos (Madrid) (ES)
(74) Representative: Carpintero Lopez, Francisco

(56) References cited:
- WO-A1-01/33226
- WO-A1-02/20832
- WO-A1-2005/086172
- WO-A1-2009/053195
- WO-A2-2007/006834
- ZHANG N-H ET AL.: "A model for the hybridization exothermic effect in label-free biodetections by a nanomechanical cantilever-DNA chip" INTERNATIONAL JOURNAL OF THERMOPHYSICS, vol. 30, 2009, pages 648-660, XP002589855
- BISWAL S L ET AL: "Nanomechanical Detection of DNA Melting on Microcantilever Surfaces" ANALYTICAL CHEMISTRY, AMERICAN CHEMICAL SOCIETY, US LNKD- DOI:10.1021/AC052171Y, vol. 78, 20 September 2006 (2006-09-20), pages 7104-7109, XP003022198 ISSN: 0003-2700
- MERTENS J ET AL: "Label-free detection of DNA hybridization based on hydration-induced tension in nucleic acid films" NATURE NANOTECHNOLOGY, NATURE PUBLISHING GROUP, LONDON, GB LNKD- DOI:10.1038/NNANO.2008.91, vol. 3, no. 5, 1 January 2008 (2008-01-01) , pages 301-307, XP002485129 ISSN: 1748-3387 [retrieved on 2008-04-20]
- BISWAL S L ET AL: "Using a Microcantilever Array for Detecting Phase Transitions and Stability of DNA", JOURNAL OF THE ASSOCIATION FOR LABORATORY AUTOMATION, ELSEVIER, vol. 11, no. 4, 1 August 2006 (2006-08-01) , pages 222-226, XP024969889, ISSN: 1535-5535, DOI: 10.1016/J.JALA.2006.06.006 [retrieved on 2006-08-01]

## Description

### FIELD OF THE INVENTION

The invention is related to the field of nucleic acid biosensors. More specifically, it relates to a method for bioanalysis of a selected type of nucleic acid molecule in a sample based on the behaviour of a gold/silicon element functionalized with nucleic acid layers when it is subjected to temperature cycles, the behaviour of the layers being different when the target molecule is in the sample (when there is hybridisation) or not. The method of the invention is also capable of discriminating the presence of single mismatches in the target molecules. The invention also relates to the system for carrying out the method of the invention.

### STATE OF THE ART

Nucleic acid biosensors are important tools in different areas such as molecular biology and functional genomics. For example, they have become more and more useful for the detection of diseases and pathogens.

In the specific field of nucleic acid biosensors, a well-known type of nucleic acid biosensor is based on the immobilization of a layer of nucleic acids (sometimes referred to as a nucleic acid probe), with a sequence chosen to specifically bind a target molecule, on a solid support. This layer is then exposed to a sample in which the target molecules (complementary nucleic acids) may be present. Usually, after exposure of the sensor surface to the sample, the sensor is carefully rinsed with different buffer solutions in order to remove non-target molecules that may have interacted with the nucleic acid probe. It is also common to use the temperature to remove this kind of non-specific interactions.

For instance, if the nucleic acid probe comprises single stranded DNA (ssDNA), the molecules that will bind to it with the highest affinity will be single stranded DNA with the complementary sequence (following the Watson-Crick rules, A with T and G with C). However, samples are usually a complex mixture of different ssDNA (or RNA) sequences that can contain regions that can be complementary to a region of the single stranded DNA probe. This gives rise to the formation of non-specific unions between the probe and non-target molecules. As the strength of these unions tends to be weaker than the unions between the probe and the target molecules, the temperature control is useful to detach the non-target molecules from the nucleic acid probe, that is, from the surface of the biosensor. The temperature is thus raised to a value somewhat lower than the "melting temperature" of the probe-and-target union, thus "detaching" or "removing" only the non-target molecules. This kind of temperature control has been found to be useful to distinguish the presence of target molecules from the presence of sequences with a single mismatch with the DNA probe.

Nowadays nucleic acid sensors are used in array formats (often referred to as DNA arrays), in such a way that thousands of nucleic acid probes are immobilized on solid support using microdispensing techniques. Hence, for example the differential gene expression between healthy cells and disease cells can be studied and the genes responsible of a disease determined.

The detection of the interaction between the nucleic acid probes on the solid support and the complementary molecules (such as the target molecules) is often performed by fluorescence microscopy. This requires a pre-treatment of the sample with fluorescent dyes.

Recently, the use of flexible microsupports such as microcantilevers has made it possible to measure the binding between complementary nucleic acids directly, without need of fluorescent markers. Thereby, time and money can be saved, and the risk for human errors in the tedious step of labelling of the sample can be reduced. In these microsupport devices, the biological interactions are measured via the bending, deformation, strain, and/or resonant properties of the flexible support.

These microsupport systems include systems based on cantilevers having a fixed end and a movable end; in these systems, it is usually the displacement and/or movement of the "free" end that is detected. However, there are also systems based on cantilevers clamped at both ends, in which the movement of the central part can be detected. Further, there are other micro- and nanomechanical structures that are movable and flexible, such as doubly clamped paddles whose "easy" direction of motion corresponds to the torsion of the paddle around the axis of the hinges that connect the paddle to a frame (basically, like a square racket fastened to a frame by two opposite handles of the racket, extending along an axis). Other known systems use membranes that are connected to a frame through two sets of hinges, which allows two angular degrees of freedom. In chemical/biological sensors based on this kind of systems, the surface of the micro- or nanomechanical element is sensitised with the receptor or "probe" molecules that selectively recognize the targeted substance. Thus, the interaction between the immobilized probe and the targeted substance on the surface of the micro- or nanomechanical element produces a change of the shape, profile, strain, stress and/or motion (vibration) of the mechanical element. This change is usually monitored by measuring the displacement of a representative part of the mechanical element (usually the free end of a singly clamped microcantilever, although it can also be the centre of a doubly clamped microcantilever, a part of a membrane sheet, etc.). This displacement can be in the order of about 1-100 nanometers (nm) and in many cases it is necessary to obtain a resolution better than 1 nm, depending on the application. For the readout of the displacement, there are several techniques such as capacitive detection, detection based on tunnel current, optical interferometry, piezoresistive readout and the optical beam deflection technique.

Examples of MMS(micro or nanomechanical systems) and microcantilever systems are disclosed in, for example:
Engel et al, "Atomic force microscopy: a powerful tool to observe biomolecules at work", Trends in Cell Biology, Volume 9, pp. 77-80 (1999*).*
P. Vettiger et al, "The millipede-more than one thousand tips for future AFM storage", IBM J. Res. Develop., Volume 44, Number 3, pp. 323-339 (2000*)*.
WO-A-2001/33226
WO-A-2003/091458
WO-A-2005/086172
WO-A-2007/006834

Often, the mechanical feature (beam displacement, vibration characteristics) is monitored in real-time during the development of the interaction between the immobilized probe molecules and the target or when the interaction is broken. A typical example is the detection of the changes in the mechanical feature while the probe is brought in contact with the sample (normally, a fluid that may contain the target molecules) and the target molecules bind to the probe. Another example is the performance of melting experiments with DNA, where a mechanical feature is observed while the temperature is raised to or beyond the temperature for the separation of certain DNA strands, whereby the detachment of the target molecules follows *(Nanomechanical Detection of DNA Melting on Microcantilever Surfaces*; S.L. Biswal, D. Raorane, A. Chaiken, H. Birecki, A. Majumdar, Analytical Chemistry 78, 7104-7109 (2006*)).*

Now, the prior art systems seem to imply certain problems. For example:
- Sometimes, the detected "change" in the mechanical feature (such as the displacement of a portion of a mechanical element) arises from other factors and not from the interaction between the receptor surface and the target molecules. For example, the change can be produced by non-specific interactions of non-target molecules with the probe, small variations in the temperature, small fluctuations in the ion concentrations in liquid sample, fluid turbulences, slow variations with time in the positions of the optical elements that detect the displacement of the mechanical element, etc.
- Also, in order to detect the change, it is often necessary to monitor the mechanical element in real time, that is, basically, from the moment in which it enters into contact with the sample, so that the change is detected while it takes place. Data regarding the feature both before and after the change must be measured in the exact same conditions, without removal of the mechanical element from the solution. Removal and replacement of the mechanical element or any other minor change in the conditions implies the loss of reference for the measurement.
- Real-time measurements imply the need to perform the optical detection of the mechanical feature while the mechanical element is immersed in liquid. Detection in liquid is technologically demanding. The mechanical response always shows drift. It is very sensitive to changes in the pressure of the liquid, small temperature differences between the background solution and the injected sample solution, instabilities at the time of the injection and delays in the response derived from the diffusion of the target molecules in the liquid.
- Also, the magnitude of the change (such as a change in curvature) can be fairly small, which obviously makes the detection an even more difficult task.
- Melting experiments are performed in liquid environment. They are based on the fact that the double helix can be separated at a defined temperature that depends on the ionic strength of the medium, the base composition and length of the DNA. This implies that subtle variations in the ionic strength, that can be uncontrollable in many cases, will bias the measurement of the melting temperature and degrade the response of the sensor.
- Many, if not all, of the prior art methods referred to above are based on the detection in liquid environments. This implies the need for microfluidic systems, to avoid the use of large amounts of precious samples. The formation of bubbles in these systems is an added difficulty during measurements.

WO-A-2009/053195 discloses a different method that allows for detection "ex-situ", that is, it does not disclose a real-time detection method. Basically, according to this method, the mechanical element is first sensitized with the receptor layer, and afterwards it is incubated with the sample solution so that the target-receptor interaction may take place. The interaction is not followed in real-time, that is, the changes in the relevant mechanical feature are not monitored while the interaction between target and probe is taking place. Instead, the detection is performed ex-situ. The signature for the detection of the target molecules is the distinct response of the reacted and the non-reacted biolayer to changes in a condition; basically, a mechanical feature (displacement, vibration, etc.) is measured for a plurality of different "values" of the condition, and the relation between the mechanical feature and the different "values" of the condition is used to determine, for example, if a target molecule is present in the sample. That is, the "relation" between the values of the condition and the measured data regarding the mechanical feature are used as a kind of "fingerprint" to detect, for example, the presence of a target molecule in the sample. WO-A-2009/053195 also mentions some examples of "conditions" that could be varied, including temperature, relative humidity, pH and ionic strength of a fluid in which the mechanical element is placed, the relative content of a mixture of gases or liquids in which the mechanical element is placed, radiation, electrical field or magnetic field. However, the only example discussed to a certain extent uses the relative humidity as the condition subjected to changes. It is not immediately clear from WO-A-2009/053195 how the other "conditions" could be used.

In what regards the relative humidity, the control of the relative humidity in the proximity of a nucleic acid microcantilever sensor makes it possible to specifically detect DNA with femtomolar sensitivity, four orders of magnitude better than with conventional DNA arrays. The origin of the bending of the cantilever is in the in-plane forces that emerge when water molecules adsorb into the highly packed nucleic acid layer. These forces are a result of a complex interaction between electrostatic forces between cantilever-anchored DNA molecules (the DNA is a highly charged molecule), van der Waals forces, hydrogen bonds and the conformational entropic elasticicy of the DNA molecules. Despite this complexity, the pattern of the cantilever bending vs the relative humidity (a relative measurement of the water adsorption in the biolayer) is extremely sensitive to the degree of hybridization and to the presence of a single base unpairing.

Now, the authors of the present invention have discovered that self assembled monolayers of single stranded and double stranded DNA show extraordinary thermal properties in temperature ranges close to room temperature (this is, at temperatures where no melting occurs). The authors have discovered that ssDNA and dsDNA layers show large negative thermal expansion coefficients in said ranges of temperature. The inventors have also observed that the expansion/contraction of the DNA layers show reversible behaviour with some hysteresis.

This discovery represents the basis of the present invention. The inventors have used these extraordinary thermal properties to develop a non-melting method for thermal characterization of DNA biolayers and especially a method for detection of nucleic acid molecules from a sample with high sensitivity.

### DESCRIPTION OF THE INVENTION

Before the description of the invention there are some terms which should be defined in order to better understand the scope of the invention.
**Nucleic acid or Nucleic acid molecule.** Any polymeric or oligomeric molecule having a backbone containing a sequence of nitrogenous bases -adenine (A), thymine (T), cytosine (C) and guanine (G). In the context of the present invention nucleic acid molecules include, *inter alia*, DNA molecules, RNA molecules, aptamers or PNA molecules
**DNA**. An organic acid and polymer composed of four nitrogenous bases -adenine, thymine, cytosine, and guanine- linked via intervening units of phosphate and the pentose sugar deoxyribose. DNA is the genetic material of most organisms and usually exists as a double-stranded molecule in which two antiparallel strands are held together by hydrogen bonds between adenine-thymine and cytosine-guanine.
**RNA**. An organic acid and polymer composed of four nitrogenous bases -adenine, uracile, cytosine, and guanine
   - linked via intervening units of phosphate and the pentose sugar ribose. RNA is a genetic material present in most organisms and usually exists as a single-stranded molecule although it may hybridize with homologous sequences of DNA or RNA by hydrogen bonds between adenine-uracile and cytosine-guanine.
**Aptamer.** A nucleic acid molecule designed in vitro to specifically interact with a ligand molecule. The specificity of an aptamer-ligand pair can be as high as that of an antibody-antigen pair.
**Peptide nucleic acid (PNA)**. An artificial kind of nucleic acid molecule where the sugar-phosphate backbone has been replaced by a peptidomimetic structure. Each monomer of PNA is composed by a molecule of N-(2-aminoethyl)glycine linked, via a methylencarbonyl unit, to a nucleobase of the group formed by A, G, C and T. PNA exhibits unique physicochemical properties, being an uncharged, achiral and relatively rigid polymer of high biological and chemical stability.
**Sample.** A liquid preparation containing the material to be bioanalised by the method of the invention.
**Target molecule**. A molecule capable of hybridizing to the nucleic acid molecules arranged in the bioactive layer with sufficient strength not to be removed after the washing for removal of unspecifically binded molecules from the receptor surface or after raising the temperature during the performance of the method of the invention. The target molecule derives its binding property from its sequence which usually is fully complementary to the sequence of the nucleic acid molecule arranged in the bioactive layer. Target molecules also comprise non-fully complementary sequences conserving the sufficient strength in their binding not to be removed with washing or temperature raising during the performance of the method. These non-fully complementary sequences normally contain no more than 1 to 3 mismatches with the DNA molecule arranged in the bioactive layer.
**Hybridization.** The process of hydrogen bonding between two complementary strands of nucleic acids such as two complementary strands of DNA or one each of DNA and RNA to form a double-stranded molecule.
**Mismatch (or despairing)**. It occurs when in at least one position of a double stranded nucleic acid, both chains have two non-complementary nucleotides that do not hybridize.
**Bioactive layer**. Nucleic acid layer immobilized on the (for example, silicon/gold) mechanical elements that provide specific detection of target molecules.
**Modified bioactive layer**. If the sample contains any target molecule, when the sample is put in contact with the nucleic acid bioactive layer, a hybridization will take place giving rise to a modified bioactive layer. A modified bioactive layer shows different properties to those of the original nucleic acid bioactive layer, in terms of, for example, elasticity, thermal expansion, wetability.
**Oligonucleotide**. A single stranded nucleic acid molecule of between 3 and 250 nucleotides long that can hybridise with a complementary nucleic acid sequence.
**Probe**. It refers to oligonucleotides of a given length comprised by specific nucleotide sequences that permit total or partial hybridization with complementary target sequences under certain conditions. In the context of this invention, the probe oligonucleotides are immobilized on silicon/gold supports to form the bioactive layers.
**Mechanical transducer element**. Movable and/or deformable part, related to the bioactive layer so that a change in a feature of the bioactive layer affects at least one detectable feature of the mechanical transducer element, for example, its position, deflection, deformation, vibration characteristics, among others. Examples of mechanical transducer elements include a cantilever, a paddle, a membrane, etc. Sometimes, the detectable feature -or change in said feature- is read by means of applying a stimulus to the electromechanical transducer element, for example by inducing vibration thereof.
**Mechanical feature**. In the context of the present invention, the "mechanical feature" of the bioactive layer is any property that can affect or influence the behaviour or characteristics of the mechanical transducer element with which it is intimately associated, so as to affect a feature of said mechanical transducer element. Examples of mechanical features are strain in, extension of, deformation in, displacement of (a portion of), rigidity of, resonant frequency of, quality factor of and expansion/contraction of said bioactive layer, among others.
**Bioanalysis**. It comprises the determination of a characteristic of the sample.
**Determination of a characteristic of the sample**. it includes the determination inter alia of the presence or not of the target nucleic acid molecule in the sample, and/or the concentration of the target nucleic acid molecule in the sample, and or the presence of any mismatch between the target nucleic acid molecule and the probe.

A first aspect of the invention relates to a method for bioanalysis of a selected type of nucleic acid molecule in a sample, comprising the steps of:
a) bringing a receptor surface of a mechanical transducer element (such as a cantilever, a paddle, a membrane, etc., as referred to above) in contact with the sample, said receptor surface comprising a bioactive layer of nucleic acids arranged to interact with a target nucleic acid molecule, so that, after contact with the sample, said bioactive layer of nucleic acids is converted into a modified bioactive layer due to interaction with the nucleic acid molecules of the sample (whereby certain characteristics of the modified bioactive layer will depend on whether or not, and/or on the extent to which, the target is present in the sample; for example, these characteristics can include the tension in said layer, such as the in-plane stress of said layer, the elastic modulus of said layer, and the thermal expansion coefficient of said layer; of course, these characteristics will also depend on the way in which the mechanical transducer element has been treated after contact with the sample, for example, on whether it remains in contact with the liquid sample or whether it has been rinsed and dried and placed in a controlled environment, for example, in a gaseous environment or in vacuum);
   characterised in that the method further comprises the steps of:
b)varying the temperature of the modified bioactive layer so as to produce a change in at least one mechanical feature of said modified bioactive layer, thereby producing a change of at least one feature of said mechanical transducer element(such as the strain in, or the mechanical deformation of, or the displacement of a portion of, or the vibrational characteristics -such as the resonant frequency and/or the quality factor- of, said mechanical transducer element);
c) measuring said feature of said mechanical transducer element to obtain data regarding said feature of said mechanical transducer element corresponding to, at least, two different temperatures of said modified bioactive layer, said temperatures being selected so as not to produce any removal(or desorption) of the target and/or detected nucleic acid molecule from the modified bioactive layer (that is, no "melting" is produced between said target sequence and the nucleic acid bioactive layer; this means that the modified bioactive layer can be kept unaltered so that, for example, the measurements can be repeated at a later stage; that is, these steps of the method can be "reversible"; for example, no unbinding, or at least no substantial or complete unbinding, between target and bioactive layer is desired, for example, no melting that "destroys" the modified bioactive layer);
d) determining, on the basis of said data, at least one characteristic of the sample (this characteristic of the sample can include, for example, the presence or not of the target in the sample, and/or the concentration of the target in the sample, and/or the presence and/or concentrations of elements not identical but structurally or functionally similar to the target; for example, if the target is a nucleic acid sequence complementary to a nucleic acid sequence of the bioactive layer, a structurally similar element could be another nucleic acid sequence with one or more nucleic acid mismatches).

This approach differs from prior art approaches in that it uses data obtained for different temperatures of the modified bioactive layer to determine one or more characteristics of the sample. That is, instead of relying on relative humidity as suggested in WO-A-2009/053195*,* and instead of using the temperature to remove the target whereby the temperature at which removal or "melting" takes place can be used to arrive at conclusions about the interactions that have occurred on the biolayer, varying temperature is used to obtain a set of data that can be compared to known "temperature profiles" of specific hybridizations or specific phenomena such as mismatches (for example while studying Single Nucleotide Polymorphisms (SNPs)), to arrive at conclusions about the sample and its contents. That is, the invention is based on "temperature profiles" or "temperature fingerprints". Thus, just as in WO-A-2009/053195*,* the analysis can be performed "ex situ" and at the time chosen by the person performing the analysis, and not necessarily when and where the interaction between the bioactive layer and the sample takes place. The use of the temperature as a varying condition, so that data can be obtained for different temperatures and thereafter be compared with known "fingerprints" or "temperature profiles" of selected target elements, opens up the door for a wide range of new applications.

Said change in a mechanical feature of said modified bioactive layer can comprise a contraction of the modified bioactive layer when the temperature is increased, and/or expansion of the modified bioactive layer when the temperature is reduced. It has been observed that for nucleic acid biolayers , a very large contraction with temperature increase exists, that varies substantially depending on the specific composition of the layer. This phenomenon has been observed for layers comprising DNA, and a similar behaviour can be expected for layers comprising other nucleic acids, such as RNA, PNA and aptamers. Thus, the expansion and contraction of the modified bioactive layer with changing temperature can be very useful for determining not only if a specific target was present in the sample, but also to determine whether, for example, associative phenomena are occurring when, for instance, nucleic acid sequences similar but not identical to a target sequence are present. The important differences in thermal contraction behaviour can make it possible to detect even single base mismatches.

The bioactive layer comprising nucleic acids can feature a (negative) temperature expansion coefficient that varies substantially with the composition of the layer, which makes it especially appropriate to use a varying temperature to obtain sets of data that can be compared to known "fingerprints" of different target nucleic acid molecules.

According to one possible implementation of this aspect of the invention, the mechanical element can be arranged, during at least steps b) and c), in a gaseous atmosphere and/or in vacuum. Due to the fact that the use of data obtained at different temperatures makes it possible to carry out the measurements "ex situ", it is no longer necessary to carry out the measurements while the mechanical element is still in contact with the liquid sample. This implies important advantages, for example, in what regards the timing of the analysis, and the place where -and the conditions under which- the analysis has to be carried out. It also implies important advantages in what regards the technology necessary to carry out the measurements since many complications arise when detection needs to be performed in liquids and/or in real-time (bubbles, thermal drifts, instabilities upon injection of sample, lack of reproducibility,...).

The method can further comprise the steps of:
- modifying the relative humidity to which the modified bioactive layer is exposed; and
- carrying out step c) so that it comprises measuring said feature of said mechanical transducer element to obtain said data regarding said feature of said mechanical transducer element corresponding to; at least, two different temperatures, for at least two different relative humidities.

It has been found also that the thermal expansion and contraction of nucleic acid layers is very dependent on the degree of hydration, which can be varied by modifying the relative humidity of the atmosphere to which the layer is subjected. Thus, by combining temperature and relative humidity the sensitivity and the reliability of the method can be increased and optimised: for example, if two different target molecules feature similar temperature profiles -that is, relations between measured data and temperature- at one relative humidity, this may not be so at all other relative humidities. Thus, and due to the relation found between the degree of hydration and the thermal expansion coefficient, the relative humidity and the temperature provide, in conjunction, a synergetically improved reliability of the system, reducing the risk for errors in the analysis.

The temperature of the modified layer can be controlled by controlling at least one feature (such as the intensity, and/or the duration of light pulses, and/or the time lapse between subsequent light pulses) of light directed onto said mechanical transducer element (for example, directly onto the bioactive layer).

In the method, an array of said mechanical transducer elements can be used, and at least some of the mechanical transducer elements of said array can be provided with bioactive layers of nucleic acids differing from bioactive layers of nucleic acids from other mechanical transducer elements of said array. In this way, in one single step large amounts of data regarding the sample can be obtained, relating to several different target nucleic acid molecules.

Another aspect of the invention relates to a method for bioanalysis of a selected type of nucleic acid molecule in a sample, comprising the steps of:
a) bringing a receptor surface of a mechanical transducer element in contact with the sample, said receptor surface comprising a bioactive layer of nucleic acids arranged to interact with a target nucleic acid molecule, so that, after contact with the sample, said bioactive layer of nucleic acids is converted into a modified bioactive layer due to interaction with the nucleic acid molecules of the sample;
   and further comprising the steps of:
b)varying the temperature of the modified bioactive layer so as to produce a change in at least one mechanical feature of said modified bioactive layer, thereby producing a change of at least one feature of said mechanical transducer element;
c) measuring said feature of said mechanical transducer element to obtain data regarding said feature of said mechanical transducer element corresponding to, at least, two different temperatures of said modified bioactive layer;
d) determining, on the basis of said data, at least one characteristic of the sample;
wherein said change in a mechanical feature of said modified layer comprises a contraction of the modified bioactive layer when the temperature is increased, and/or an expansion of the modified bioactive layer when the temperature is reduced.

A further aspect of the invention relates to a method for bioanalysis of a selected type of nucleic acid molecule in a sample, comprising the step of:
a) bringing a receptor surface of a mechanical transducer element in contact with the sample, said receptor surface comprising a bioactive layer of nucleic acids arranged to interact with a target nucleic acid molecule, so that, after contact with the sample, said bioactive layer of nucleic acids is converted into a modified bioactive layer due to interaction with the nucleic acid molecules of the sample;
   and further comprising the steps of:
b)varying the temperature of the modified bioactive layer so as to produce a change in at least one mechanical feature of said modified bioactive layer, thereby producing a change of at least one feature of said mechanical transducer element;
c) measuring said feature of said mechanical transducer element to obtain data regarding said feature of said mechanical transducer element corresponding to, at least, two different temperatures of said modified bioactive layer;
d) determining, on the basis of said data, at least one characteristic of the sample;
wherein the method further comprising the steps of:
- modifying the relative humidity to which the modified bioactive layer is exposed; and
- carrying out step c) so that it comprises measuring said feature of said mechanical transducer element to obtain said data regarding said feature of said mechanical transducer element corresponding to, at least, two different temperatures, for at least two different relative humidities.

A further aspect of the invention relates to a system for surface inspection arranged to detect relative displacement and/or vibration characteristics of a plurality of elements forming part of a mechanical structure for bioanalysis of a sample, said system comprising:
- a light source arranged to generate at least one light beam;
- a position sensitive detector arranged to receive the light beam when reflected off the mechanical structure and to produce at least one output signal in response to receipt of said light beam;
- an electronic control system;
wherein
the system further comprises a sub-system for varying and controlling the temperature of said plurality of elements.

The mechanical structure can be arranged within a chamber, and the system can further comprise a sub-system for varying and controlling relative humidity within the chamber.

For example, a system such as the one disclosed in WO-A-2009/053195 could be used, if complemented with a suitable sub-system for temperature control.

The sub-system for varying and controlling the temperature of said plurality of elements can comprise at least one light source arranged to direct light onto said plurality of elements, and means for controlling at least one characteristic of the light emitted by said light source in order to control the temperature of said plurality of elements. This light source can be the same as the one generating the previously mentioned light beam, but can also be a different one. The characteristic of the light that is controlled can be, for example, the intensity of the light, and/or the duration of light pulses, and/or the time lapse between subsequent light pulses. Using light as the heat source instead of using means such as Peltier elements can provide for a simple and practical control of the temperature.

The system can be configured to carry out the method of any of the above described aspects of the invention (the system can be configured to carry out the method by incorporating, for example, a suitable software).

### BRIEF DESCRIPTION OF THE DRAWINGS

To complete the description and in order to provide for a better understanding of the invention, a set of drawings is provided. Said drawings form an integral part of the description and illustrate a preferred embodiment and examples of the invention, which should not be interpreted as restricting the scope of the invention, but just as examples of how the invention can be embodied. The drawings comprise the following figures:

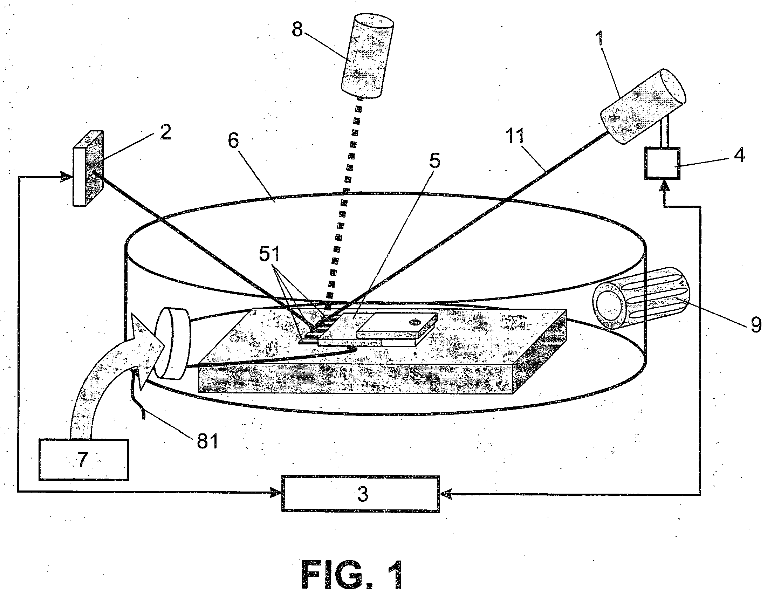

**Figure 1****:** Schematically illustrates a system according with one possible embodiment of the invention.

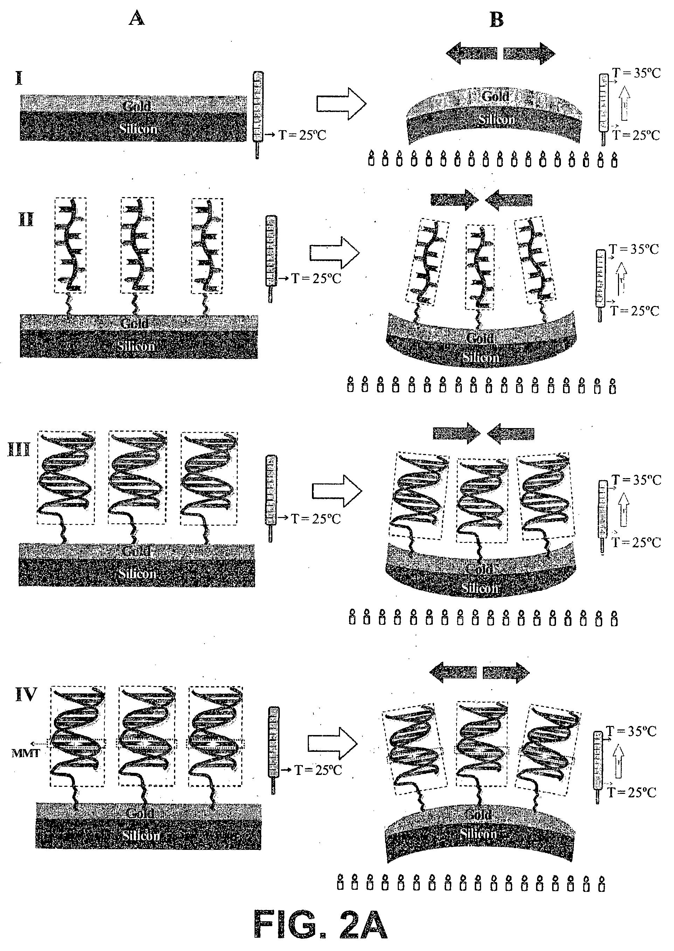

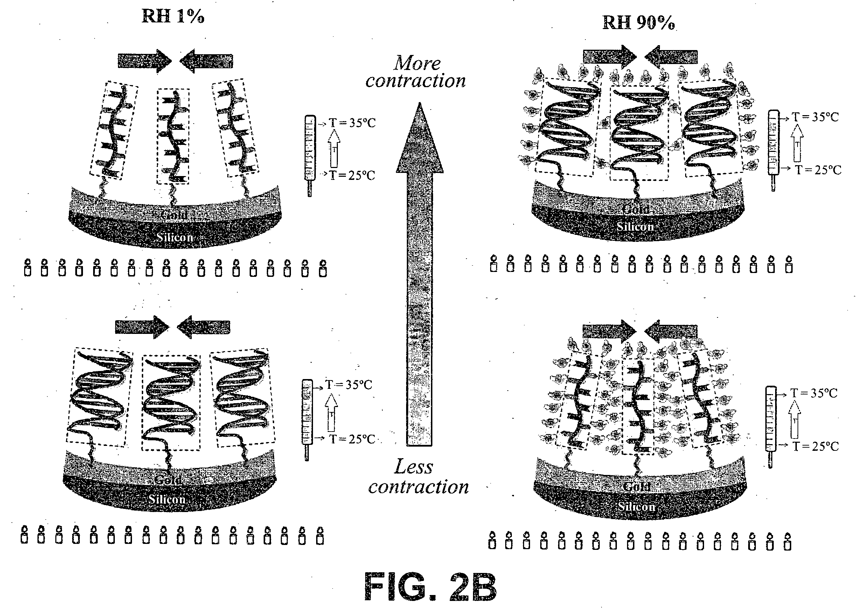

**Figure 2****: A.** Schematically illustrates the effect of temperature on a bare silicon element (a cantilever) with a gold layer (I) and on such element with nucleic acid layers such as ssDNA (II), dsDNA (III) and dsDNA with a single mismatch (IV) immobilized on the gold layer. **B**. Schematically illustrates the combined effect of temperature and relative humidity on single stranded and double stranded nucleic acid layers.

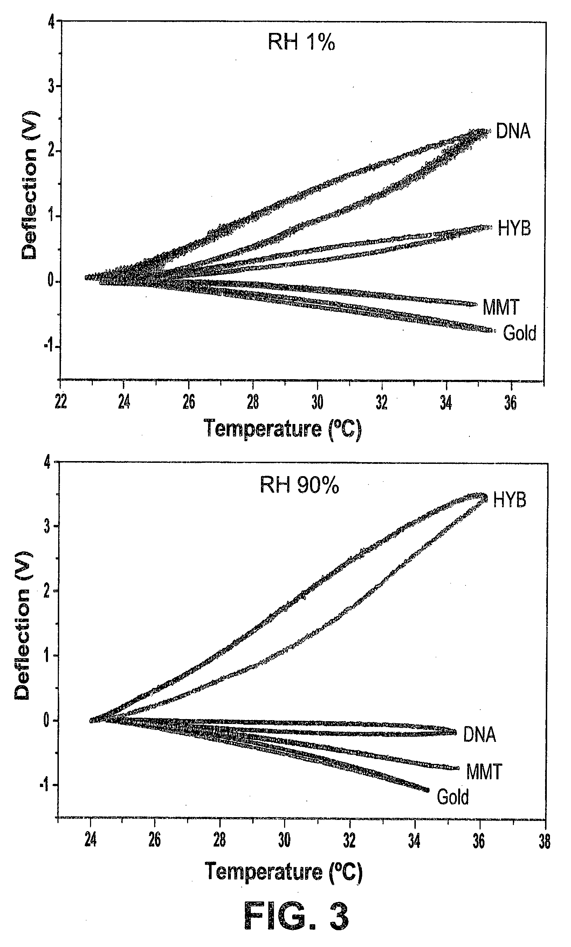

**Figure 3****:** Shows the response and hysteresis of a ssDNA sensitized (DNA) cantilever while subjected to a temperature cycle, compared to the response and hysteresis of the cantilever with dsDNA (HYB) (after full complementary probe-target hybridization) subjected to the same temperature cycle. The response is always reversible, this indicates that the biolayer is not being destroyed or permanently changed during the experiment. The response is different when the environment is kept at a high relative humidity with respect to an environment of low relative humidity. The figure also shows the deflection variation and hysteresis for a mismatched DNA target sequence (MMT) during a temperature cycle and the deformation of a gold coated cantilever with changes in temperature (Gold). **A**. The larger thermal expansion coefficient of the gold layer with respect to the silicon film induces a negative bending of the microcantilever (towards the uncoated silicon side). If the silicon/gold microcantilever is coated with a ssDNA layer then the deflection is positive (towards the gold coated side) when increasing the temperature, meaning the ssDNA layer contracts with increasing temperature (DNA). When the ssDNA sensitised microcantilever is incubated in a solution with a complementary DNA strand, we find that for the hybridised layer (HYB) the thermal behaviour is qualitatively the same, but the hybridized layer contracts significantly less with temperature, providing with a new biosensing parameter/signature of the hybridization. All these experiments where performed in a dry atmosphere (1% RH). **B**. The same experiments as in **A**. where conducted in a high humidity air environment (RH 90%). It is observed that in this case the dsDNA layer after hybridization contracts significantly more than the ssDNA layer, due to the different content in water of the biolayers.

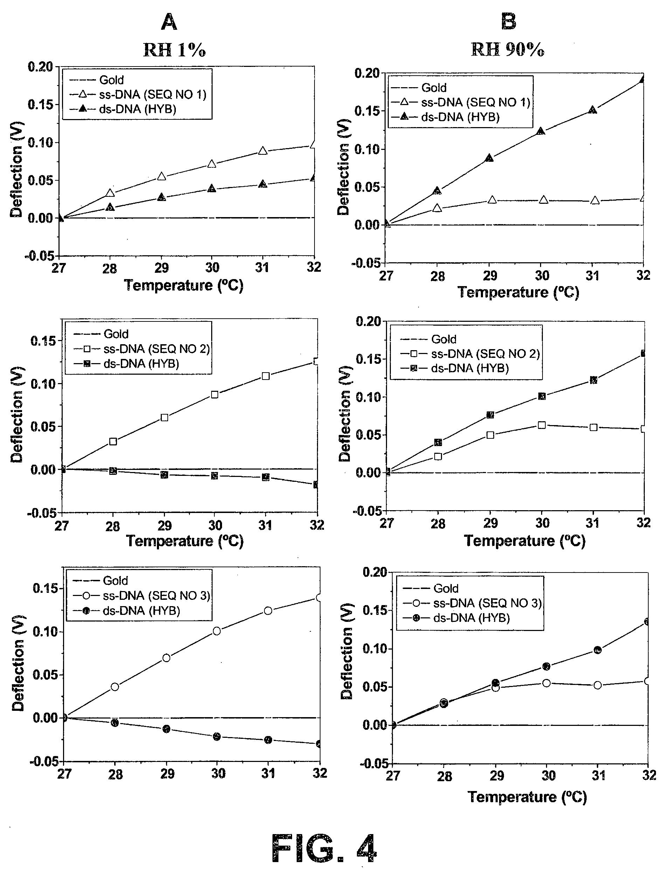

**FIGURE 4****:** Cantilever deflection vs. temperature for a gold coated cantilever after ssDNA Self Assembled Monolayer formation (open symbols) and after hybridization (filled symbols). **A.** The measurements were carried out in dry conditions, r.h.< 1%. **B**. The measurements were carried out in humid conditions, r.h.≈90%. The response of three thiolated ssDNA sequences is shown: 16-mer 5'-HS-CTA CCT TTT TTT TCT G-3' (SEQ ID NO 1), 25 mer 5'-HS- ACT GCA ACC AGT TTC CTC TTG GGT G-3'(SEQ ID NO 2) and 25 mer 5'-HS- ACT GCA ACC AGT CTC CTC TTG GGT G-3' (SEQ ID NO 3). The deflection of the bare gold coated cantilever has been substracted from all measurements to eliminate any effect related to the bimetallic effect.

### DESCRIPTION OF A PREFERRED EMBODIMENT OF THE INVENTION, AND EXAMPLES

A system in accordance with a preferred embodiment of the invention is illustrated in figure 1. It can be based on a system such as the one disclosed in WO-A-2009/053195, but adapted to also control temperature. More specifically, figure 1 illustrates a system for surface inspection arranged to detect relative displacement and/or vibration characteristics of a plurality of points of a plurality of cantilevers 51 forming part of a mechanical structure 5. The system comprises:
a light source 1 arranged to generate at least one light beam 11;
a position sensitive detector 2 arranged to receive the light beam when reflected off the mechanical structure 5 and to produce at least one output signal in response to receipt of said light beam;
an electronic control system 3; and
scan means 4 for relative displacement of said light beam with respect to the mechanical structure 5 so as to scan said mechanical structure with the light beam, following instructions from the electronic control system 3.

The electronic control system 3 can be arranged to control the scan means 4 so as to displace the light beam along a first trajectory, so as to detect a plurality of subsequent reference positions along said first trajectory, and the electronic control system can be operatively associated with the position sensitive detector 2 so as to determine said reference positions as a result of an analysis of at least one output signal from said position sensitive detector 2, as described in WO-A-2009/053195. Also, the control system can be further arranged to control the scan means 4 so as to displace the light beam along the mechanical structure along a plurality of second trajectories, each of said second trajectories being associated with one of said reference positions, as also described in WO-A-2009/053195.

The mechanical structure 5, or at least the cantilevers 51, are arranged within a chamber 6, and the system comprises a sub-system 7 for varying and controlling relative humidity within the chamber 6, and a sub-system 8 for varying and controlling the temperature of said plurality of elements 51, under the control of the electronic control system. The sub-system 8 for varying and controlling the temperature of said plurality of elements 51 can comprise at least one light source arranged to direct light onto said plurality of elements, and means for controlling at least one characteristic (such as intensity, etc.) of the light emitted by said light source in order to control the temperature of said plurality of elements. A temperature sensor can be provided to help to control the temperature 81.

Thus, this system can provide for checking the "finger-print" of a sample -or of the "modified layer" after incubation with a given solution- by obtaining measured data regarding the mechanical elements not only at different temperatures, but also at different relative humidities, as outlined above. The system can also comprise further components, such as a hygrometer 9, etc.

### EXAMPLE

### I - Description of the technique

The experimental set-up used in the experiments is illustrated in Figure 1. DNA molecules were anchored to the cantilevers in order to monitor how the surface stress (deflection of the cantilever) changed. Single stranded DNA probes were immobilized on a gold-coated side of microcantilevers via a thiol linker. Monocrystalline silicon microcantilever arrays 400 µm long, 100 µm wide and 1.0 µm thick were coated by sputtering evaporation with a 20 nm gold layer on top of a 2 nm adhesion layer of chromium. Freshly coated microcantilevers were incubated with 1 µM of the thiol-modified single stranded DNA (ssDNA) diluted in PBS buffer (137 mM NaCl, 2.7 mM KCI, 8 mM Na₂HPO₄ 2 mM KH₂PO₄; pH=7.5) at 25ºC, 24-48 hours in order to immobilize a densely packed DNA layer. Afterwards, the cantilevers were vigorously rinsed in PBS buffer first and Milli-Q water afterwards to discard unspecific interactions, and then dried under a stream of dry nitrogen gas. After the long immobilization time ssDNA formed a highly packed layer with a surface density of the order of 10¹³ cm⁻², as determined by X-ray photoelectron spectroscopy (XPS) experiments.

The experiments were performed in controlled gas/humidity environment. The hybridization of the sensitized cantilever with the complementary sequence was performed in PBS at 24ºC, at a concentration of 1 µM during 2-4 hours.

Using this experimental approach, it was discovered that self assembled monolayers of single stranded and double stranded DNA show extraordinary thermal properties. Particularly, ssDNA and dsDNA layers show a large contraction with temperature in a temperature range close to room temperature (see figure 3). Although thermal contraction with increasing temperature has been measured for a variety of materials, including polymer films, this is the first time that a measurement of the thermal expansion/contraction of DNA layers is performed. It was also observed that the expansion/contraction of the DNA layers show a reversible behaviour with some hysteresis (figure 3).

The thermal expansion/contraction of the Self Assembled Monolayer of ssDNA and dsDNA was measured by immobilizing the biolayer on a gold coated silicon microcantilever. Then the expansion/contraction of the layer could be detected by measuring how the tension in the monolayer changed as a result of a temperature change. The approach relied on the microcantilever bending in a quantity that depended on the tension in the monolayer. The tension translated in a deformation of the thin silicon microcantilever was measured by a scanning laser system.

These studies provide interesting insight about the thermal properties of these ultrathin films. DNA films are particularly relevant for technological applications and are experimentally easy to control for developing fundamental applications regarding the effect of confinement between film-air and film-substrate interfaces on nanosoft materials. Importantly, these measurements are performed in a temperature range which is far from the melting temperature for the dsDNA as it is therefore a non-destructive method.

One application of the recording of this thermal expansion/contraction of DNA films is in the field of biosensors. It was found that the thermal expansion/contraction of DNA films changes dramatically when the monolayer interacts with either non-complementary, complementary or single mismatched single-stranded DNA targets.

Distinct deflection behaviour upon temperature variation when experiments were performed at a relative humidity of 1% or for 90% relative humidity was also observed. This effect is attributed to the different content in water of the distinct biolayers depending on the relative humidity. The double signature (at high and low humidity) increases the reliability of a biosensing method based on this principle. The contraction is higher in the dry state than in the humid state for ssDNA. More interestingly, after hybridization, the DNA film stills contract on heating, but contraction behaviour with the humidity conditions is opposite to that of the ssDNA film. The hybridized ssDNA film significantly contracts with temperature more in humid environment than in dry conditions. Figures 2A and 2B illustrate this behaviour that is experimentally demonstrated by figure 3.

The comparison of the deflection response to temperature variation of gold coated cantilevers without any immobilized biolayer and that of functionalized or hybridized cantilevers allows to rule out the effect of the thermal expansion of gold (bimetallic effect).

The sequence of experiments in the assays were:
i) Measurement of the deflection induced by the changes in temperature close to room temperature in gold coated cantilevers. Temperature was ramped up and down again to the initial value.
ii) Measurement of the deflection induced by the changes in temperature close to room temperature in gold coated cantilevers immobilized with thiol-modified ssDNA. Temperature was ramped up and down again to the initial value.
iii) Exposure of the functionalized cantilever to a solution of the nucleic acid sample that contained: a) the fully complementary target ssDNA molecule; b) a ssDNA target with one mismatch;
   c) a non complementary ssDNA (negative control); then the cantilevers were rinsed and dried and
iv) Measurement of the deflection as the system undergoes a second temperature cycle at a higher humidity

The immobilized sequences are: 5'-HS-CTA CCT TTT TTT TCT G-3', (SEQ ID NO 1) ,5'-HS- ACT GCA ACC AGT TTC CTC TTG GGT G-3' (SEQ ID NO 2), 5'-HS- ACT GCA ACC AGT CTC CTC TTG GGT G-3'(SEQ ID NO 3).

The fully complementary ssDNA to SEQ ID NO 1 showed the sequence 5'-CAGAAAAAAAAGGTAG-3'(SEQ ID NO 4), whereas the mismatching sequence was a single T/T mismatch near the central position of the duplex SEQ ID NO 5(sequence 5'-CAGAAAATAAAGGTAG-3'). The non-complementary ssDNA (negative control) had the sequence SEQ ID NO 6(5'-AGCTTCCGTACTCGAT-3'). The fully complementary ssDNA to SEQ ID NO 2 showed the sequence 5'-CACCCAAGAGGAAACTGGTTGCAGT-3'(SEQ ID NO 7) and the fully complementary molecule to SEQ ID NO 3 showed the sequence 5'-CACCCAAGAGGAGACTGGTTGCAGT-3'(SEQ ID NO 8).

During the experiments it was not observed any non-reversible change in the biolayer, therefore, melting of the DNA layer was disregarded. The DNA melting would necessarily give place to a non-reversible bending of the cantilever (reference: Using a Microcantilever Array for Detecting Phase Transitions and Stability of DNA Journal of the Association for Laboratory Automation, Volume 11, Issue 4, Pages 222-226, S. Biswal, D. Raorane, A. Chaiken, A. Majumdar).

### I/- Effect of DNA sequence and sequence length

The thermal response of the following sequences was studied:
- 16-mer 5'-HS-CTACCTTTTTTTTCTG-3', (SEQ ID NO 1)
- 25-mer 5'-HS-ACTGCAACCAGTTTCCTCTTGGGT G-3' (SEQ ID NO 2)
- 25-mer 5'-HS-ACTGCAACCAGTCTCCTCTTGGGTG-3'(SEQ ID NO 3)
in order to determine the effect of sequence length and base composition in the thermal contraction effect.

The results showed that thermal expansion/contraction behaviour depends only quantitatively on base sequence and sequence length while the qualitative behaviour remains the same. Qualitatively, hybridization is characterized by a decrease of the magnitude of thermal contraction when compared to the response of the non-hybridized ssDNA in dry conditions and an increase of this magnitude at high humidity, for all the sequences studied. The slope of the curves depicted in figure 4 (numerical values shown below in table 1), representing the degree of bending with temperature, is indicative of the magnitude of this contraction. For the ssDNA layers, the values remain in the same range with only slight variations between shorter and longer sequences. These results imply that the fingerprint followed in these experiments for ssDNA layers is of application to any base sequence composition and sequence length. Nevertheless, contraction of the hybridized DNA layer at low relative humidity for the longer sequences (SEQ ID NO 2 and SEQ ID NO 3) is below the noise in the system. In this case, although hybridization could be detected as a decrease in the contraction of the modified biolayer, the follow up of the double signature (at low and high humidity) may be especially useful. The contribution to the deflection response of the thermal expansion of the metallic layer (bimetallic effect) has been subtracted in all cases.

**Table 1. Slopes from linear fits of data obtained for a temperature increase from 27 ºC to 32 ºC.**

| | RH 1% | | RH 90% | |
|---|---|---|---|---|
| | ss-DNA | ds-DNA | ss-DNA | ds-DNA |
| SEQ ID NO 1 | 0.019 ± 0.001 | 0.010 ± 0.001 | 0.006 ± 0.002 | 0.037 ± 0.001 |
| SEQ ID NO 2 | 0.025 ± 0.001 | -0.003 ± 0.001 | 0.012 ± 0.003 | 0.030 ± 0.002 |
| SEQ ID NO 3 | 0.028 ± 0.002 | -0.005 ± 0.001 | 0.010 ± 0.002 | 0.026 ± 0.001 |

### III- Sensitivity and specificity of the system

Among the different mechanical parameters that could be measured, the deflection in a temperature cycle was chosen. This was found to be very sensitive to the DNA form, i.e., non hybridized ssDNA, mismatched or hybridized dsDNA. Therefore, this was chosen to be the measurement parameter of the biosensor. The combination of this parameter with that of relative humidity provided improved reliability as the different water content of the ssDNA layer and that of the dsDNA layer provided a double signature for the discrimination of the hybridization event. Since the hybridization was performed in all experiments at 24ºC, significant variations were not expected in the hybridization yield of the fully complementary and single-mismatched sequences (theoretical calculations show that the discriminatory temperature for the used targets should be expected to lie in the range 34-43ºC). The mismatched sequence was clearly detected in these experiments due to its qualitatively distinct behaviour or temperature fingerprint.

Figure 3 shows the response of the biolayer after incubation with a mismatched sequence (SEQ ID NO 5), labelled MMT in figure 3. The layer contracted significantly less than for the layer hybridized with the fully complementary sequence (SEQ ID NO 4), labelled as HYB, as well as significantly less than the ssDNA layer after incubation with the control sequence (SEQ ID NO 6), labelled as DNA in figure 3, where no hybridization occurred.

This behaviour implies that hybridization with the mismatched sequence occurred but that mismatched layers do not show the same contraction fingerprint as fully complementary layers or highly packed ssDNA layers. Furthermore, the mismatched sequence contraction is not affected by the level of hydration, this is, the relative humidity of the chamber in the experiments, as can be seen from comparison between graphs in figure 3A with graphs in figure 3B.

Without being bound to any theory, the inventors believe that the mechanism for the mismatch discrimination is due to the stabilization of a mismatch-induced "discontinuity" in the duplex by hydrogen bonding. This "discontinuity" in the layer implies a different tension pattern in the biolayer that allows mismatch discrimination. The rationale for this finding is that these nucleic acid monolayers cooperatively respond, acting as a highly responsive membrane, to temperature variations, and the responsivity is highly modulated by the connectivity between the ssDNA or dsDNA probes. The connectivity of the ssDNA membrane is, in the case of mismatched sequences, changed by the hybridization. Some studies point at percolation and entropic effects with microcantilevers in line with this assumption (Ref DOI: 10.1002/adma.200801344, Physics of Nanomechanical Biosensing on Cantilever Arrays, Maria L. Sushko, John H. Harding, Alexander L. Shluger, Rachel A. McKendry, Moyu Watari, Adv. Mater. 2008, 20, 3848-3853; REF Nanomechanical detection of antibiotic-mucopeptide binding in a model for superbug drug resistance, J. W. Ndieyira et al., Nature Nanotechnology 3,691 (2008)).

As a consequence of the previous finding, a relevant feature of the method developed is that, contrarily to what happens in DNA microarrays and other current biosensors, the differential behaviour of the mismatched target does not rely on its tendency to de-hybridize at an optimized, fine-tuned working temperature, but on the particular intrinsic property, this is, thermal expansion/contraction behaviour of a biolayer, or temperature fingerprint, that contains the mismatch induced "discontinuities". Experiments are here always far below the melting temperature.

In conclusion, the intermolecular forces in monolayers of nucleic acids provide the layer with distinct thermal expansion properties that critically depend on the subtle molecular characteristics of the biolayer. The high molecular density attained by the long immobilization times used makes that hybridization of about one target molecule per million of probes produces a dramatic effect in the thermal expansion/contraction. This principle also allows the discrimination of single mutations. Unprecedented sensitivity achieved by the monitoring of the thermal expansion/contraction of DNA layers is expected. These experiments are the starting point for rapid and straightforward genotyping or SNP mapping close to room temperature, without need of sample amplification and labelling. Moreover, the use of temperature variations in a range close to room temperature allows the simplification of the biosensing equipment, as the need for accurate control of gas environments is no longer necessary. Even experiments combining temperature and relative humidity dependent information can be conducted at two relative humidities, "high" and "low", but an accurate control of the actual humidity is no longer needed.

## Claims

1. Method for bioanalysis of a selected type of nucleic acid molecule in a sample, comprising the step of:
a) bringing a receptor surface of a mechanical transducer element in contact with the sample, said receptor surface comprising a bioactive layer of nucleic acids arranged to interact with a target nucleic acid molecule, so that, after contact with the sample, said bioactive layer of nucleic acids is converted into a modified bioactive layer due to interaction with the nucleic acid molecules of the sample;
**characterised in that** the method further comprises the steps of:
b)varying the temperature of the modified bioactive layer so as to produce a change in at least one mechanical feature of said modified bioactive layer, thereby producing a change of at least one feature of said mechanical transducer element;
c) measuring said feature of said mechanical transducer element to obtain data regarding said feature of said mechanical transducer element corresponding to, at least, two different temperatures of said modified bioactive layer, said temperatures being selected so as not to produce any removal of the target nucleic acid molecule from the modified bioactive layer;
d) determining, on the basis of said data, at least one characteristic of the sample
e) modifying the relative humidity to which the modified bioactive layer is exposed; and
f) carrying out step c) so that it comprises measuring said feature of said mechanical transducer element to obtain said data regarding said feature of said mechanical transducer element corresponding to, at least, two different temperatures, for at least two different relative humidities.

2. Method according to claim 1 where the nucleic acids present in the sample or making up the bioactive layer are aptamers, DNA, RNA, or PNA molecules.

3. Method according to any of the preceding claims, wherein said change in a mechanical feature of said modified bioactive layer comprises a contraction of the modified bioactive layer when the temperature is increased, and/or an expansion of the modified bioactive layer when the temperature is reduced.

4. Method according to any of the preceding claims, wherein, during at least steps b) and c), the mechanical element is arranged in a gaseous atmosphere and/or in vacuum.

5. Method according to any of the preceding claims where the characteristic of the sample determined in step d) is the presence or absence of the target nucleic acid molecule in the sample, and/or the concentration of the target nucleic acid molecule in the sample, and/or the presence and/or concentrations of any target molecule having at least one mismatch with regard to the nucleic acid molecules arranged in the bioactive layer.

6. A method according to any of the preceding claims, wherein the temperature of the modified bioactive layer is controlled by controlling at least one feature of light directed onto said mechanical element.

7. Method according to claim 1, wherein an array of said mechanical transducer elements is used, at least some of the mechanical transducer elements of said array being provided with bioactive layers of nucleic acids differing from bioactive layers of nucleic acids of other mechanical transducer elements of said array.

## Patentansprüche

1. Verfahren zur Bioanalyse eines ausgewählten Typs von Nucleinsäuremolekül in einer Probe, umfassend die Schritte:
a) Bringen einer Rezeptorfläche eines mechanischen Wandlerelements in Kontakt mit der Probe, wobei die Rezeptorfläche eine bioaktive Schicht von Nucleinsäuren umfasst, die so angeordnet sind, dass sie mit einem Zielnucleinsäuremolekül in Wechselwirkung treten, so dass die bioaktive Schicht von Nucleinsäuren nach Kontakt mit der Probe aufgrund der Wechselwirkung mit den Nucleinsäuremolekülen der Probe in eine modifizierte bioaktive Schicht umgewandelt wird;
**dadurch gekennzeichnet, dass** das Verfahren weiterhin die Schritte umfasst:
b) Variieren der Temperatur der modifizierten bioaktiven Schicht unter Hervorbringen einer Veränderung in wenigstens einem mechanischen Merkmal der modifizierten bioaktiven Schicht, wodurch eine Änderung wenigstens eines Merkmals des mechanischen Wandlerelements hervorgebracht wird;
c) Messen des Merkmals des mechanischen Wandlerelements, um Daten bezüglich des Merkmals des mechanischen Wandlerelements zu erhalten, die wenigstens zwei verschiedenen Temperaturen der modifizierten bioaktiven Schicht entsprechen, wobei die Temperaturen so gewählt sind, dass die zu keiner Entfernung des Zielnucleinsäuremoleküls aus der modifizierten bioaktiven Schicht führen;
d) Bestimmen wenigstens einer Eigenschaft der Probe auf der Basis dieser Daten;
e) Modifizieren der relativen Feuchtigkeit, der die modifizierte bioaktive Schicht ausgesetzt ist; und
f) Durchführen von Schritt c) in einer solchen Weise, dass er das Messen des Merkmals des mechanischen Wandlerelements umfasst, um Daten bezüglich des Merkmals des mechanischen Wandlerelements, die wenigstens zwei verschiedenen Temperaturen entsprechen, für wenigstens zwei verschiedene relative Feuchtigkeiten zu erhalten.

2. Verfahren gemäß Anspruch 1, wobei die in der Probe vorhandenen oder die bioaktive Schicht bildenden Nucleinsäuren Aptamere, DNA-, RNA- oder PNA-Moleküle sind.

3. Verfahren gemäß einem der vorstehenden Ansprüche, wobei die Veränderung in einem mechanischen Merkmal der modifizierten bioaktiven Schicht eine Kontraktion der modifizierten bioaktiven Schicht bei Temperaturerhöhung und/oder eine Expansion der modifizierten bioaktiven Schicht bei Temperatursenkung umfasst.

4. Verfahren gemäß einem der vorstehenden Ansprüche, wobei das mechanische Element wenigstens während der Schritte b) und c) in einer Gasatmosphäre und/oder im Vakuum angeordnet ist.

5. Verfahren gemäß einem der vorstehenden Ansprüche, wobei die in Schritt d) bestimmte Eigenschaft der Probe die Anwesenheit oder Abwesenheit des Zielnucleinsäuremoleküls in der Probe und/oder die Konzentration des Zielnucleinsäuremoleküls in der Probe und/oder die Anwesenheit und/oder die Konzentrationen eines beliebigen Zielmoleküls, das wenigstens eine Fehlpaarung mit den in der bioaktiven Schicht angeordneten Nucleinsäuremolekülen aufweist, ist.

6. Verfahren gemäß einem der vorstehenden Ansprüche, wobei die Temperatur der modifizierten bioaktiven Schicht dadurch gesteuert wird, dass man wenigstens ein Merkmal von auf das mechanische Element gerichtetem Licht steuert.

7. Verfahren gemäß Anspruch 1, wobei ein Array der mechanischen Wandlerelemente verwendet wird, wobei wenigstens einige der mechanischen Wandlerelemente des Arrays mit bioaktiven Schichten von Nucleinsäuren versehen sind, die sich von bioaktiven Schichten von Nucleinsäuren anderer mechanischer Wandlerelemente des Arrays unterscheiden.

## Revendications

1. Procédé pour la bioanalyse d'un type sélectionné de molécule d'acide nucléique dans un échantillon, comprenant l'étape consistant à :
a) mettre une surface de récepteur d'un élément transducteur mécanique en contact avec l'échantillon, ladite surface de récepteur comprenant une couche bioactive d'acides nucléiques disposés pour interagir avec une molécule d'acide nucléique cible, de sorte que, après le contact avec l'échantillon, ladite couche bioactive d'acides nucléiques est convertie en une couche bioactive modifiée en raison de l'interaction avec les molécules d'acide nucléique de l'échantillon ;
**caractérisé en ce que** le procédé comprend en outre les étapes consistant à :
b) faire varier la température de la couche bioactive modifiée de manière à produire un changement dans au moins une caractéristique mécanique de ladite couche bioactive modifiée, produisant ainsi un changement d'au moins une caractéristique dudit élément transducteur mécanique ;
c) mesurer ladite caractéristique dudit élément transducteur mécanique afin d'obtenir des données relatives à ladite caractéristique dudit élément transducteur mécanique correspondant à, au moins, deux températures différentes de ladite couche bioactive modifiée, lesdites températures étant sélectionnées de manière à faire en sorte que la molécule d'acide nucléique cible ne soit pas éliminée de la couche bioactive modifiée ;
d) déterminer, sur la base desdites données, au moins une caractéristique de l'échantillon ;
e) modifier l'humidité relative à laquelle la couche bioactive modifiée est exposée ; et
f) réaliser l'étape c) de sorte qu'elle comprend la mesure de ladite caractéristique dudit élément transducteur mécanique afin d'obtenir lesdites données relatives à ladite caractéristique dudit élément transducteur mécanique correspondant à, au moins, deux températures différentes, pour au moins deux humidités relatives différentes.

2. Procédé selon la revendication 1, dans lequel les acides nucléiques présents dans l'échantillon ou constituant la couche bioactive sont des aptamères, des molécules d'ADN, d'ARN ou d'APN.

3. Procédé selon l'une quelconque des revendications précédentes, dans lequel ledit changement dans une caractéristique mécanique de ladite couche bioactive modifiée comprend une contraction de la couche bioactive modifiée lorsque la température est augmentée, et/ou une dilatation de la couche bioactive modifiée lorsque la température est réduite.

4. Procédé selon l'une quelconque des revendications précédentes, dans lequel, pendant au moins les étapes b) et c), l'élément mécanique est disposé dans une atmosphère gazeuse et/ou sous vide.

5. Procédé selon l'une quelconque des revendications précédentes, dans lequel la caractéristique de l'échantillon déterminée à l'étape d) est la présence ou l'absence de la molécule d'acide nucléique cible dans l'échantillon, et/ou la concentration de la molécule d'acide nucléique cible dans l'échantillon, et/ou la présence et/ou les concentrations de n'importe quelle molécule cible ayant au moins un mésappariement en ce qui concerne les molécules d'acide nucléique disposées dans la couche bioactive.

6. Procédé selon l'une quelconque des revendications précédentes, dans lequel la température de la couche bioactive modifiée est régulée en régulant au moins une caractéristique de lumière dirigée sur ledit élément mécanique.

7. Procédé selon la revendication 1, dans lequel un groupe desdits éléments transducteurs mécaniques est utilisé, au moins certains des éléments transducteurs mécaniques dudit groupe étant dotés de couches bioactives d'acides nucléiques qui diffèrent des couches bioactives d'acides nucléiques des autres éléments transducteurs mécaniques dudit groupe.


### SEQUENCE LISTING

<110> CONSEJO SUPERIOR DE INVESTIGACIONES CIENTIFICAS
<120> METHOD FOR THE BIOANALYSIS OF NUCLEIC ACID MOLECULES IN A SAMPLE AND BIOSENSOR FOR ITS IMPLEMENTATION
<130> PRE 20090189
<160> 8
<170> PatentIn version 3.3
<210> 1
   <211> 16
   <212> DNA
   <213> Artificial
<220>
   <223> contains a thiol group in the 5' end
<400> 1
   ctaccttttt tttctg 16
<210> 2
   <211> 25
   <212> DNA
   <213> Artificial
<220>
   <223> contains a thiol group in the 5' end
<400> 2
   actgcaacca gtttcctctt gggtg 25
<210> 3
   <211> 25
   <212> DNA
   <213> Artificial
<220>
   <223> contains a thiol group in the 5' end
<400> 3
   actgcaacca gtctcctctt gggtg 25
<210> 4
   <211> 16
   <212> DNA
   <213> Artificial
<220>
   <223> fully complementary sequence to SEQ ID NO 1
<400> 4
   cagaaaaaaa aggtag 16
<210> 5
   <211> 16
   <212> DNA
   <213> Artificial
<220>
   <223> single mismatched sequence to SEQ ID NO 1
<400> 5
   cagaaaataa aggtag 16
<210> 6
   <211> 16
   <212> DNA
   <213> Artificial
<220>
   <223> non-complementary sequence to SEQ ID NO 1, negative control
<400> 6
   agcttccgta ctcgat 16
<210> 7
   <211> 25
   <212> DNA
   <213> Artificial
<220>
   <223> Fully complementary sequence to SEQ ID NO 2
<400> 7
   cacccaagag gaaactggtt gcagt 25
<210> 8
   <211> 25
   <212> DNA
   <213> Artificial
<220>
   <223> Fully complementary sequence to SEQ ID NO 3
<400> 8
   cacccaagag gagactggtt gcagt 25